# EUROPÄISCHE PATENTSCHRIFT

(11) **EP 0 909 821 B1**
(45) Veröffentlichungstag und Bekanntmachung des Hinweises auf die Patenterteilung: **24.05.2006**
(21) Anmeldenummer: 98118031.8
(22) Anmeldetag: 23.09.1998
(51) Int. Cl.: C12N 15/55, C12N 15/10, C12N 9/18, C12P 7/42, C12P 41/00

(54) **Verfahren zur Änderung der Substratspezifität von Enzymen**
Engineering enzymes having altered substrate specificity
Manipulation d'enzymes presentant une spécificité changée pour le substrat

(30) Priorität: 02.10.1997 DE 19743683
(43) Veröffentlichungstag der Anmeldung: 21.04.1999
(73) Patentinhaber: BASF Aktiengesellschaft, 67056 Ludwigshafen (DE)
(72) Erfinder: Bornscheuer, Uwe, Dr., 70569 Stuttgart (DE); Meyer, Hartmut Herrmann, Prof. Dr., 30419 Hannover (DE); Altenbuchner, Josef, Dr., 71154 Nufringen (DE)

(56) Entgegenhaltungen:
- WO-A-87/05050
- WO-A-97/07202
- GREENER A ET AL.: "XL1-Red: A Highly Efficient Random Mutagenesis Strain" 1994 , STRATEGIES IN MOLECULAR BIOLOGY VOL. 7, PAGES 32-34 XP000985105 * das ganze Dokument *
- GREENER ALAN ET AL: "An efficient random mutagenesis technique using an E. coli mutator strain." 1996 , METHODS IN MOLECULAR BIOLOGY, VOL. 57, PAGE(S) 375-385 , 1996 HUMANA PRESS INC. SUITE 808, 999 RIVERVIEW DRIVE, TOTOWA, NEW JERSEY 07512, USA XP000984480 ISBN: 0-89603-332-5 * das ganze Dokument *
- REETZ M. T. ET AL.: "Creation of enantioselective biocatalysts for organic chemistry by in vitro evolution" ANGEW. CHEM. INT. ED. ENGL., Bd. 36, Nr. 24, 1997, Seiten 2830-2832, XP002087468
- NISHIYA Y. AND IMANAKA T.: "Alteration of substrate specificity and optimum pH of sarcosine oxidase by random and site-directed mutagenesis" APPL. ENVIRON. MICROBIOL, Bd. 60, Nr. 11, November 1994 (1994-11), Seiten 4213-4215, XP001021379
- SILMAN N. J. ET AL.: "Directed evolution of amidase in Methylophilus methylotrophus; purification and properties of amidases from wild-type and mutant strains" JOURNAL OF GENERAL MICROBIOLOGY, Bd. 137, 1991, Seiten 169-178, XP001018388
- BEUVE A. AND DANCHIN A.: "From adenylate cyclase to guanylate cyclase Mutational analysis of a change in substrate specificity" JOURNAL OF MOLECULAR BIOLOGY, Bd. 225, Nr. 4, 1992, Seiten 933-938, XP001018343
- BORNSCHEUER U T ET AL: "Directed evolution of an esterase for the stereoselective resolution of a key intermediate in the synthesis of epothilones" BIOTECHNOLOGY AND BIOENGINEERING - COMBINATORIAL CHEMISTRY, WILEY, NEW YORK, NY, US, Bd. 58, Nr. 5, 5. Juni 1998 (1998-06-05), Seiten 554-559, XP002144278 ISSN: 0006-3592
- PANAITE D M ET AL: "CHARACTERIZATION OF MUTANTS OF THE 6'-N-ACETYLTRANSFERASE ENCODED BY THE MULTIRESISTANCE TRANSPOSON TN1331: EFFECT OF PHE171-TO-LEU171 AND TYR80-TO-CYS80 SUBSTITUTIONS" PLASMID, NEW YORK,NY, US, Bd. 39, 1998, Seiten 123-133, XP000984506 ISSN: 0147-619X
- KUCHNER O ET AL: "Directed evolution of enzyme catalysts" TRENDS IN BIOTECHNOLOGY, ELSEVIER PUBLICATIONS, CAMBRIDGE, GB, Bd. 15, Nr. 12, 1. Dezember 1997 (1997-12-01), Seiten 523-530, XP004097432 ISSN: 0167-7799

## Beschreibung

Die Erfindung betrifft ein Verfahren zur Änderung der Substratspezifität von Enzymen.

Häufig steht der Synthesechemiker in der Praxis vor dem Problem, daß er leicht und einfach durchführbare Synthesewege zur Herstellung einer chemischen Verbindung nicht beschreiten kann, da dieser Weg zu Verbindungen führen würde, bei denen beispielsweise die Abspaltung einer benötigten Schutzgruppe nicht mehr möglich ist, da sonst das synthetisierte Molekül zerstört würde. Um dieses Problem zu lösen, muß er häufig einen umständlicheren, zeitaufwendigeren Syntheseweg einschlagen.

Enzyme spalten chemische Bindungen unter milden Bedingungen. Sie können deshalb in einigen Fällen zur Lösung derartiger Syntheseprobleme verwendet werden, d.h. eine Schutzgruppe kann beispielsweise unter milden Bedingungen ohne Zerstörung anderer Bindungen und damit des Moleküls enzymatisch abgespalten werden. Enzyme ermöglichen so im Labor einen leichten und raschen Zugang zu den gewünschten Substanzen. Häufig reicht die enzymatische Aktivität und/oder Stabilität für eine technische Nutzung der Enzyme jedoch nicht aus, so daß die chemischen Synthesen, obwohl sie über eine größere Zahl von Synthesestufen durchgeführt werden müssen, trotzdem kostengünstiger sind und deshalb technisch realisiert werden.

Zur Verbesserung der enzymatischen Aktivität und/oder Stabilität wurden deshalb eine Vielzahl von Arbeiten unternommen. Dabei wurden unterschiedliche Wege verfolgt.

Über die sogenannte "site directed mutagenesis" läßt sich die Stabilität und/oder enzymatische Aktivität der Enzyme sehr gezielt verbessern. Nachteilig bei der "site directed mutagenesis" von Enzymen ist, daß bei dieser Methode viel Wissen über die Struktur und Funktion der Enzyme aus Röntgenstrukturanalysen, aus dem Modelling, aus Vergleichen mit anderen Enzymen gleicher oder ähnlicher Spezifität vorhanden sein muß. Darüber hinaus muß die Sequenz der Strukturgene bekannt sein, um überhaupt eine zielgerichtete Verbesserung der Enzyme zu ermöglichen. In der Regel liegen diese Informationen nicht, noch nicht oder nur zum Teil vor, so daß in diesen Fällen diese Methode meist nicht zum gewünschten Erfolg führt, da nicht klar ist in welchen Bereichen das Enzym verändert werden muß. Liegen nur wenige Informationen über die enzymatische Aktivität vor, so sind Methoden, die zur Verbesserung des Enzyms eine Zufallsstrategie verfolgen, zu bevorzugen, wobei diese Methoden trotzdem so zielgerichtet wie möglich sein sollten.

So wird von Spee et al. (Nucleic Acids Research, Vol. 21, No. 3, 1993: 777 - 778) eine PCR-Methode unter Verwendung von dITP zur zufälligen Mutagenese des nisZ-Gens von Lactococcus lactis beschrieben.

Die von Spee et al. beschriebene Methode wurde von Rellos et al. (Protein Expr. Purif., 5, 1994 : 270 - 277) weiter verbessert. Rellos et al. beschreiben die Mutagenese des Alkoholdehydrogenase-2-Gens von Zymomonas mobilis mit Hilfe einer PCR-Methode ohne Verwendung von dITP, da die Limitierung eines Nucleotids für die Erhöhung der Mutationsrate und damit für den gewünschten Erfolg völlig ausreichend ist.

In WO 87/05050 wird eine Erfindung auf dem Gebiet der Waschmittelenzyme beschrieben. Die beschriebene Erfindung befasst sich mit der Mutation von Proteasen speziell von Substilisin und Proteinase K zur Erhöhung ihrer Stabilität in der Waschflotte. Sowohl Subtilisin als auch Proteinase K gehören zu den unspezifische Serinproteasen. Unter anderem wird für die Mutagenese der Stamm E. coli MutD offenbart. Von Nachteil bei der Verwendung von Proteasen in der Waschflotte ist, daß sie eine mangelnde thermische Stabilität haben und in der Waschlösung durch Detergenzien inaktiviert werden.

In WO 97/07202 wird eine weitere Erfindung auf dem Gebiet der Waschmittelenzyme offenbart. Ziel der wiedergegebenen Arbeiten ist die Mutagenese zur Erhöhung der Stabilität von lipolytischen Enzymen in der Waschflotte. Dies wird über eine gesteigerte Aktivität der Enzyme erzielt, die durch eine Mutagenese und anschließender Selektion erreicht wird. Für die Mutagenese wird in WO 97/07202 erneut der E. coli Mutatorstamm MutD (Fowler et al. 1974) offenbart.

Von Reetz et al. (Angew. Chem. Int. Ed. Engl. 1997, 36, No. 24, p. 2830 - 2832) wird ein Verfahren zur Erzeugung eines enantionselektiven Biokatalysators anhand der Lipase von Pseudomonas aeruginosa mit Hilfe der "error-prone polymerase chain reaction" beschrieben.

Die Verwendung einer "in vitro" Rekombinationstechnik für die molekulare Evolution wird von Stemmer (Proc. Natl. Acad. Sci. USA, Vol. 91, 1994: 10747 - 10751) beschrieben. Durch diese Rekombinationstechnik ließ sich die enzymatische Funktion des lacZ-Gens aus zwei mutierten und inaktivierten lacZ-Genen wieder herstellen.

Von Moore et al. (Nature Biotechnology Vol. 14, 1996: 458 - 467) wird die Kombination der PCR- und Rekombinationsmethode zur Steigerung der enzymatischen Aktivität einer Esterase gegenüber einem para-Nitrobenzylester beschrieben.

Von Nachteil bei den genannten Methoden ist, daß zur Erzeugung der Mutationen die DNA "in vitro" mit Enzymen wie der Taq-Polymerase und/oder Restriktionsenzymen und/oder Oligonukleotiden behandelt werden muß und die verschiedenen potentiellen Mutanten einzeln weiter behandelt und getestet werden müssen. Diese Methoden sind nur zur Mutagenese relativ kleiner DNA-Bereiche geeignet.

Ein weiterer Weg zur Mutagenese von Enzymen wird von Greener et al. in Methods in Molecular Biology (Vol. 57, 1996: 375 - 385) beschrieben. Greener et al. verwendet den speziellen Escherichia coli Stamm XL1-Red zur Erzeugung von Escherichia coli Mutanten, die eine erhöhte Antibiotikaresistenz aufweisen. Diese erhöhte Antibiotikaresistenz ist auf eine erhöhte Kopienzahl des Plasmids pBR322, das für eine β-Lactamase kodiert, zurückzuführen. Auch zur Erzeugung auxotropher Mutanten und zur Erhöhung der Enzymaktivität kann dieser Escherichia coli-Stamm verwendet werden.

Nachteil aller genannten Mutagenese-Methoden ist, daß nur vorhandene enzymatische Aktivitäten optimiert werden können. Sind neue enzymatische Reaktionen d.h. sind neue Substratspezifitäten der Enzyme gefragt, beispielsweise zur Spaltung eines neuen Substrats, so muß nach dieser neuen enzymatischen Aktivität zunächst in der belebten Natur in einem aufwendigen Screening gesucht werden. Anschließend muß das Enzym dann in der Regel noch weiter optimiert werden.

Es wäre deshalb wünschenswert eine Methode zu besitzen, die es ermöglicht neue enzymatische Aktivitäten zu generieren, das heißt die die Substratspezifität von Enzymen verändern kann.

Es war deshalb Aufgabe der vorliegenden Erfindung eine neue breitanwendbare Methode zu entwickeln, die die oben genannten Nachteile nicht aufweist und die die Substratspezifität von Enzymen rasch und einfach ändern kann

Diese Aufgabe wurde durch das erfindungsgemäße Verfahren zur Änderung der Substratspezifität von Enzymen, dadurch gekennzeichnet, daß man folgende Arbeitsschritte durchführt:
a) Einbringen einer DNA, die eine Kopie des für das Enzym kodierenden Gens enthält, in den Escherichia coli Stamm XL1 Red oder in ein funktionelles Derivat, das die Genmarker relAl, mutS, mutT und mutD5 trägt,
b) Inkubation des transformierten Escherichia coli Stammes XL1 Red oder seines funktionellen Derivat zur Erzeugung von Mutationen im Enzymgen,
c) Weitergabe der mutierten DNA aus dem Stamm XL1 Red oder seinem funktionellen Derivat an einen Mikroorganismus, der keine behindernde Enzymaktivität aufweist,
d) Inkubation dieses Mikroorganismus zur Detektion der Enzymaktivität auf oder in mindestens einem Selektionsmedium, das mindestens ein Enzymsubstrat, das die Erkennung einer geänderten Substratspezifität des Enzyms ermöglicht, und ggf. weitere Indikatorsubstanzen enthält,
e) Auswahl der Mikroorganismen, die eine Änderung der Substratspezifität zeigen,
wobei die Änderung der Substratspezifität zu einer stereoselektiven enzymatischen Aktivität führt, gelöst.

Unter Änderung der Substratspezifität im erfindungsgemäßen Verfahren ist zu verstehen, daß die Enzyme nach Durchlaufen des Verfahrens in der Lage sind Substrate umzusetzen, die sie vorher nicht umsetzen konnten, da die Affinität des Enzyms zum Substrat zu gering war (= hoher K_{M}-Wert) und/oder die Enzyme eine zu geringe katalytische Aktivität (= k_{cat}) aufwiesen. In diesen Fällen ist der Quotient k_{cac}/K_{M}Null oder nahezu Null, das heißt es liegt keine Katalyse vor. Durch die Änderung der Substratspezifität wird der K_{M}-Wert verringert oder der k_{cat}-Wert erhöht oder beides, das heißt der Quotient k_{cat}/K_{M} wird größer Null. Es kommt zu einer katalytischen Reaktion. Das Enzym setzt das neue Substrat nach Mutagenese um.

Prinzipiell kann die Substratspezifität aller Enzyme verändert werden, bevorzugt wird im erfindungsgemäßen Verfahren die Substratspezifität von Hydrolasen verändert. Hydrolasen bilden im Nomenklatursystem der IUB die 3. Enzym-Klasse (= 3..). Hydrolasen sind im erfindungsgemäßen Verfahren bevorzugt, da für sie in der Regel eine einfache Nachweisreaktion besteht und sie vielfach in technischen Synthesen Anwendung finden. Besonders bevorzugt wird die Substratspezifität von Hydrolasen ausgewählt aus der Gruppe bestehend aus Proteasen, Lipasen, Phospholipasen, Esterasen, Phosphatasen, Amidasen, Nitrilasen, Etherhydrolasen, Peroxidasen und Glykosidasen verändert, ganz besonders bevorzugt werden Lipasen, Esterasen, Nitrilasen oder Phytasen.

Nach Änderung der Substratspezifität im erfindungsgemäßen Verfahren kann die Enzymreaktion mit einer Selektivität bei chiralen Ausgangsverbindungen erfolgen, das heißt die Reaktion führt zu optisch aktiven Produkten. Bevorzugt werden Änderungen, die zu selektiven Änderungen in der Substratspezifität und damit zu stereoselektiven Reaktionen führen.

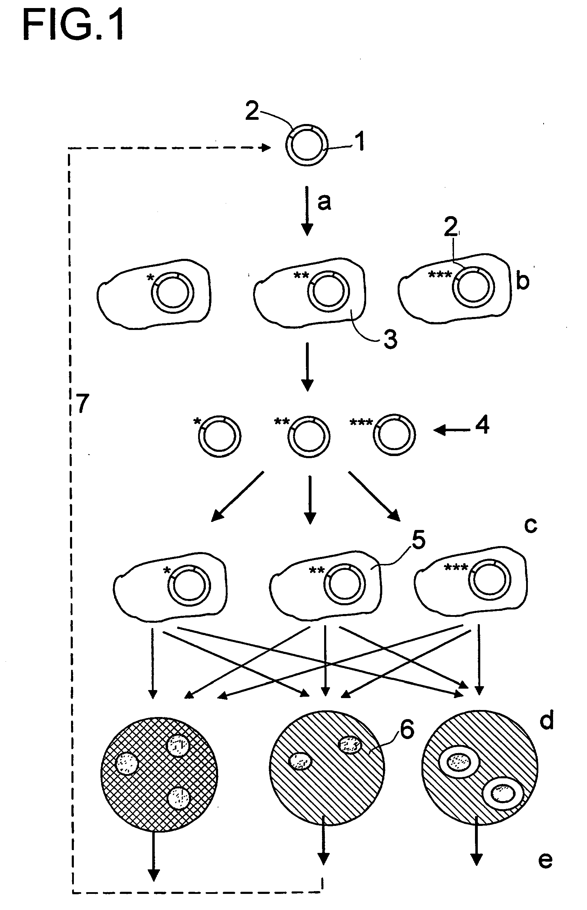

Im erfindungsgemäßen Verfahren sind alle dem Fachmann bekannten Methoden zum Einbringen von DNA in Mikroorganismen geeignet (Verfahrensschritt a, Figur 1). Die DNA kann dabei über Phagen, über Transformation oder über Konjugation in den Stamm Escherichia coli XL1 Red oder eines funktionellen Derivats dieses Stammes eingebracht werden. Als vorteilhaft geeignete Phagen seien alle temperenten Phagen wie Lambda oder Mu genannt. Verfahren wie diese Phagen-DNA in die entsprechenden Mikroorganismen gebracht werden, sind dem Fachmann wohl bekannt (siehe Microbiology, Third Edition, Eds. Davis, B.D., Dulbecco, R., Eisen, H.N. and Ginsberg, H.S., Harper International Edition, 1980). Bei der Konjugation kann die DNA direkt übertragen werden, das heißt sie ist auf dem konjugationsvermittelnden Plasmid lokalisiert wie beispielsweise auf dem F-Faktor, oder sie wird bei der Konjugation über einen komobilisierbaren Vektor übertragen. Auch diese Methoden sind dem Fachmann bekannt. Als bevorzugte Methode sei das Einbringen der DNA über Transformation genannt (Winnacker, E.L., From Genes to Clones, VCH, 1987: 126 - 127, Williams et al., Ann. Rev. Gen., Vol. 14, 1980: 41 - 76). In der Literatur sind zahllose dem Fachmann bekannte Methoden zur Transformation von Mikroorganismen beschrieben, dabei werden verschiedenste Reagenzien wie PEG (Chung et al. Proc. Natl. Acad. Sci. USA, Vol. 86, 1989: 2172 - 2175), CaCl₂ (Mandel et al., J. Mol. Biol. Vol. 53, 1970: 159 - 162), Dimethylsulfoxid, Hexamincobalt und Dithiothreitol in Gegenwart von Mono- oder divalenten Kationen (Hanahan D., J. Mol. Biol., 166, 1983: 557 - 580) oder Elektroporation benutzt (siehe DNA Cloning 1, Ed. Clover et al., IRL Press, 1995, Hanahan et al., Technics for Transformation of E. coli, page 1-36, ISBN 0199634769).

Für die Transformation lassen sich alle üblichen Vektoren verwenden. Üblicherweise werden Vektoren, die sich in Escherichia coli replizieren lassen, verwendet. Soll als Selektionsmikroorganismus, der die Detektion der Enzymaktivität ermöglicht, ein Mikroorganismus eines anderen Reiches beispielsweise Pilze wie Aspergillus, Ashbya oder Beauveria oder Hefen wie Saccharomyces, Candida oder Pichia, einer anderen Familie beispielsweise Actinomycetales oder einer anderen Gattung Pseudomonas, Streptomyces, Rhodococcus oder Bacillus verwendet werden, so werden vorteilhafterweise sogenannte "shuttle vectoren" verwendet, die sich in beiden Mikroorganismen replizieren lassen, da eine Umklonierung der DNA so nicht erforderlich ist.

Als Vektoren seien die folgenden Plasmide beispielhaft genannt pLG338, pACYC184, pBR322, pUC18, pUC19, pKC30, pRep4, pPLc236, pMBL24, pLG200, pUR290, pIN-III²²³-B1, λgtl1 oder pBdCI. Weitere Vektoren sind dem Fachmann wohl bekannt und können beispielsweise aus dem Buch Cloning Vectors (Eds. Pouwels P. H. et al. Elsevier, Amsterdam-New York-Oxford, 1985 , ISBN 0 444 904018) entnommen werden.

Für das erfindungsgemäße Verfahren zur Erzeugung der Mutationen (Verfahrensschritt b, Figur 1) besonders geeignet ist der Escherichia coli Stamm XL1 Red (= Epicurian coli XL-1 Red), der von der Firma Stratagene La Jolla, CA vertrieben wird. Er trägt die folgenden Genmarker: Δ[mcrA]183, Δ[mcrCB-hsdSMR-mrr]173, endA1, supE44, thi-1, gyrA96, relAl, mutS, mutT, mutD5, lac. MutS ist eine Mutation im sog. "mismatch repair pathway", mutT ist eine Mutation im "oxo-dGTP repair pathway" und mutD5 ist eine Mutation in der 3'-5'Exonucleaseuntereinheit der DNA Polymerase III. Kompetente Zellen dieses Stammes können von Stratagene unter der Bestellnummer 200129 gekauft werden. Unter einem funktionellen Derivat dieses Stammes sind Escherichia coli Stämme zu verstehen, die folgende Genmarker relAl, muts, mutT und mutD5 enthalten. Diese Genmarker führen in den Organismen zu einer deutlich erhöhten Mutationsrate. Sie sollten deshalb auf Agarplatten oder in einem Anzuchtsmedium nicht zu lange inkubiert werden, da sie sonst ihre Vitalität verlieren.

Zur Detektion der veränderten Substratspezifität (= Mutationen im verwendeten Enzym) kann die DNA zunächst vorteilhafterweise, im Falle das Vektoren verwendet wurden, aus dem E. coli Stamm XL1 Red oder seinem funktionellen Derivat isoliert und in einen Mikroorganismus, der keine entsprechende Enzymaktivität aufweist, eingebracht (Verfahrensschritt c, Figur 1) werden. Wird beispielsweise eine Esterase in diese Selektionsorganismen eingebracht, so dürfen diese Mikroorganismen keine Esteraseaktivität aufweisen, die den für die Selektion auf eine Änderung der Substratspezifität der Esterase verwendeten Ester spalten. Andere Esteraktivitäten in diesem Organismus stören die Selektion nicht. Das Einbringen der DNA kann wie oben beschrieben über Phagen bzw. Viren, über Konjugation oder über Transformation erfolgen. Im Falle des Einbringens über Phagen bzw. Viren oder über Konjugation ist ein spezielles Isolieren der DNA nicht erforderlich. Die DNA kann direkt über Konjugation oder über den Phagen bzw. Virus in den für die Selektion verwendeten Mikroorganismus eingebracht werden. Die Weitergabe der DNA erfolgt also in diesen Fällen ohne ein Isolieren der DNA, im Falle der Verwendung von Vektoren über eine Transformation. Auch die DNA der Mikroorganismen, die eine veränderte Substratspezifität nach Selektion aufweisen, kann ohne Isolierung über Konjugation oder Phagen bzw. Viren oder über Transformation in den Stamm E. coli XL1 Red oder einem funktionellen Derivat für eine weitere Selektionsrunde eingebracht werden (= Figur 1, gestrichelte Linie). Auf diese Weise kann das erfindungsgemäße Verfahren ein oder mehrmals in Folge durchlaufen werden. Die DNA wird dabei aus den E. coli Stämmen XL1 Red oder dessen funktionellen Derivaten an die Selektionsorganismen weitergegeben und gelangt schließlich für eine neue Selektionsrunde wieder zurück in die E. coli Stämme.

Als Selektionsmikroorganismen im erfindungsgemäßen verfahren sind prinzipiell alle prokaryontischen oder eukaryontischen Mikroorganismen geeignet, wobei sie keine enzymatische Aktivität aufweisen dürfen, die die Selektion behindern könnte. Darunter ist zu verstehen, daß entweder keine enzymatische Aktivität in den Mikroorganismen vorliegt, nach der gesucht wird, das heißt das oder die zur Selektion der veränderten Substratspezifität verwendeten Substrate durch die Enzyme der Selektionsmikroorganismus nicht umgesetzt werden, oder das nur eine geringe entsprechende enzymatische Aktivität in den Mikroorganismen vorliegt, die eine Selektion noch ermöglicht. Als Mikroorganismen kommen für das erfindungsgemäße Verfahren vorteilhaft grampositive oder gramnegative Bakterien, Pilze oder Hefen in Frage. Bevorzugt werden grampositive Bakterien wie Bacillus, Rhodococcus, Streptomyces oder Nocardia oder gramnegative Bakterien wie Salmonella, Pseudomonas oder Escherichia verwendet. Ganz besonders bevorzugt werden Escherichia coli Stämme verwendet. Wobei die Gattung und Art oder die Zugehörigkeit zu einer Familie oder einem Reich des für die Selektion verwendeten Mikroorganismus von untergeordneter Rolle ist, soweit er eine Selektion der veränderten Substratspezifität ermöglicht.

Für die Selektion der veränderten Substratspezifität werden die Mikroorganismen zur Detektion der Enzymaktivität auf oder in mindestens einem Selektionsmedium, das mindestens ein Enzymsubstrat, das die Erkennung einer geänderten Substratspezifität des Enzyms ermöglicht, inkubiert (Figur 1, Verfahrensschritt d). Dieses Selektionsmedium kann gegebenenfalls weitere Indikatorsubstanzen enthalten, die eine bessere Erkennung der gewünschten Änderung ermöglichen. Solche zusätzlichen Indikatorsubstanzen können beispielsweise pH-Indikatoren sein.

Figur 1 gibt die einzelnen Schritte des Verfahrens beispielhaft unter Verwendung eines Vektors (1) wieder. Verfahrensschritt a zeigt das Einbringen der DNA (2) über den Vektor in den Stamm Escherichia coli XL1 Red oder in ein funktionelles Derivat (3) dieses Stammes. Die DNA des Enzyms wird in diesen Organismen mutiert [Sterne in Figur 1 deuten beispielhaft die Mutationen in der DNA (2) schematisch an]. Die mutierten Vektoren (4) werden anschließend wieder aus dem Stamm Escherichia coli XL1 Red oder seinen funktionellen Derivaten isoliert und anschließend direkt oder nach einer Lagerung in die Selektionsorganismen (5) transformiert (Verfahrensschritt c). Diese Organismen werden schließlich auf mindestens einem Selektionsmedium (6) ausplattiert und so die veränderte Enzymsubstratspezifität über beispielsweise einen Wachstumsassay und/oder einen visuellen Assay identifiziert (Verfahrensschritt d). Positive Klone, die eine veränderte Substratspezifität aufweisen, werden schließlich ausgesucht und das mutierte für das veränderte Enzym kodierende Gen kann isoliert werden (Verfahrensschritt e). Das Verfahren kann unter Verwendung des mutierten Gens mehrfach wiederholt werden [Figur 1, gestrichelte Linie (7)].

Im erfindungsgemäßen Verfahren werden die verwendeten Mikroorganismen, das heißt der Escherichia coli XL1 Red Stamm sowie seine funktionellen Derivate sowie die verwendeten Selektionsorganismen in einem Medium, das das Wachstum dieser Organismen ermöglicht angezüchtet. Dieses Medium kann ein synthetisches oder ein natürliches Medium sein. Je nach Organismus werden dem Fachmann bekannte Medien verwendet. Für das Wachstum der Mikroorganismen enthalten die verwendeten Medien eine Kohlenstoffquelle, eine Stickstoffquelle, anorganische Salze und gegebenenfalls geringe Mengen an Vitamine und Spurenelemente.

Vorteilhafte Kohlenstoffquellen sind beispielsweise Zucker wie Mono-, Di- oder Polysaccharide wie Glucose, Fructose, Mannose, Xylose, Galaktose, Ribose, Sorbose, Ribulose, Lactose, Maltose, Saccharose, Raffinose, Stärke oder Cellulose, komplexe Zuckerquellen wie Melasse, Zuckerphosphate wie Fructose-1,6-bisphosphat, Zuckeralkohole wie Mannit, Polyole wie Glycerin, Alkohole wie Methanol oder Ethanol, Carbonsäuren wie Citronensäure, Milchsäure oder Essigsäure, Fette wie Sojaöl oder Rapsöl, Aminosäuren wie Glutaminsäure oder Asparaginsäure oder Aminozucker, die auch gleichzeitig als Stickstoffquelle verwendet werden können.

Vorteilhafte Stickstoffquellen sind organische oder anorganische Stickstoffverbindungen oder Materialien, die diese Verbindungen enthalten. Beispiele sind Ammoniumsalze wie NH₄Cl oder (NH₄)₂SO₄, Nitrate, Harnstoff, oder komplexe Stickstoffquellen wie Maisquellwasser, Bierhefeautolysat, Sojabohnenmehl, Weizengluten, Hefeextrakt, Fleischextrakt, Caseinhydrolysat, Hefe oder Kartoffelprotein, die häufig auch gleichzeitig als Stickstoffquelle dienen können.

Beispiele für anorganische Salze sind die Salze von Calcium, Magnesium, Natrium, Mangan, Kalium, Zink, Kupfer und Eisen. Als Anion dieser Salze sind besonders das Chlor-, Sulfat- und Phosphation zu nennen.

Gegebenenfalls werden dem Nährmedium weitere Wachstumsfaktoren zugesetzt, wie beispielsweise Vitamine oder Wachstumsförderer wie Riboflavin, Thiamin, Folsäure, Nicotinsäure, Pantothenat oder Pyridoxin, Aminosäuren wie Alanin, Cystein, Asparagin, Asparaginsäure, Glutamin, Serin, Methionin oder Lysin, Carbonsäuren wie Citronensäure, Ameisensäure, Pimelinsäure oder Milchsäure, oder Substanzen wie Dithiothreitol.

Zur Stabilisierung der DNA, die in den Vektoren oder Phagen enthalten ist, in den Zellen können gegebenenfalls Antibiotika dem Medium zugesetzt werden.

Das Mischungsverhältnis der genannten Nährstoffe hängt von der Art der Inkubation (= Fermentation) ab und wird im Einzelfall festgelegt. Die Mediumkomponenten können alle zu Beginn der Fermentation vorgelegt werden, nachdem sie falls erforderlich getrennt sterilisiert oder gemeinsam sterilisiert wurden, oder aber je nach Bedarf während der Inkubation nachgegeben werden.

Plattenmedien sind gegenüber Flüssigmedien bevorzugt, da sie eine leichtere Detektion der gewünschten Änderung der Substratspezifität ermöglichen. Wichtig ist, daß keine Mediumskomponenten verwendet werden, die die Detektion der veränderten Enzymaktivität stören könnten.

Die Züchtungsbedingungen werden so festgelegt, daß die Organismen (= Escherichia coli Stamm XL1 Red und Selektionsorganismen) optimal wachsen und daß die bestmöglichen Ausbeuten erreicht werden. Bevorzugte Züchtungstemperaturen liegen bei 15 °C bis 40 °C. Besonders vorteilhaft sind Temperaturen zwischen 25 °C und 37 °C. Vorzugsweise wird der pH-Wert in einem Bereich von 3 bis 9 festgehalten. Besonders vorteilhaft sind pH-Werte zwischen 5 und 8. Im allgemeinen ist eine Inkubationsdauer von 1 bis 240 Stunden, bevorzugt von 5 bis 170 Stunden, besonders bevorzugt von 10 bis 120 Stunden ausreichend, in einzelnen Fällen können zur Mutagenese oder Detektion auch längere Inkubationsdauern erforderlich sein.

Die veränderte Substratspezifität kann nach Identifizierung der entsprechenden Klone nochmals vorteilhaft in einem "in vitro" Assay nachkontrolliert werden.

### Beispiele:

Für die Synthese des Macrolidantibiotikums Epothilon A (siehe Formel I) erwiesen sich die 3-Hydroxyester [siehe Formel II, (1) und (3)] in der retrosynthetischen Betrachtung als günstige Ausgangsverbindung.

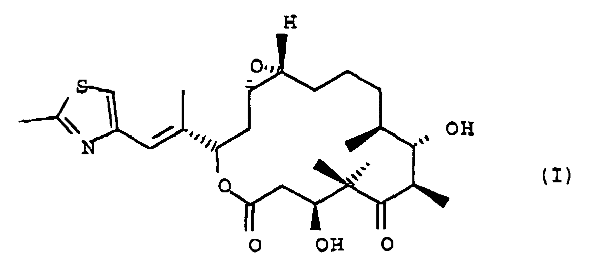

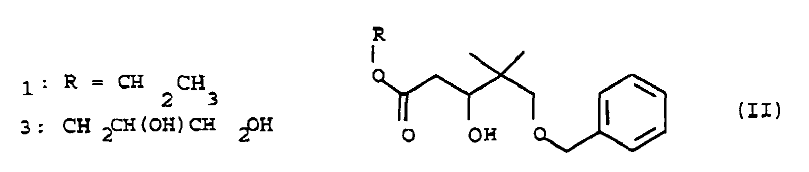

Für die optisch aktive Herstellung der Verbindungen wurde ein enzymatischer Weg mit Hilfe von Esterasen bzw. Lipasen eingeschlagen. Überraschenderweise zeigte keine der verwendeten 18 Lipasen und zwei Esterasen eine enzymatische Aktivität gegenüber den 3-Hydroxyestern (siehe Tabelle I). Weder in der Hydrolyserichtung, die in einem Phosphatpuffer durchgeführt wurde, noch in der Syntheserichtung, die mit Vinylacetat in Toluol durchgeführt wurde, konnte eine enzymatische Aktivität nachgewiesen werden.

**Tabelle I: Getestete Lipasen und Esterasen - Hydrolyse des E-thylesters (II, 1)**

| **Enzym aus** | **Hersteller** |
|---|---|
| Pseudomonas cepacia (= PS) | Amano, Nagoya, Japan |
| Pseudomonas cepacia (= AH) | Amano, Nagoya, Japan |
| Acylase (ACS) | Amano, Nagoya, Japan |
| Rhizopus delamar (D) | Amano, Nagoya, Japan |
| Rhizopus javanicus (F-AP 15) | Amano, Nagoya, Japan |
| Candida rugosa (AY) | Amano, Nagoya, Japan |
| Mucor javanicus (M) | Amano, Nagoya, Japan |
| Penicillium roquefortii (R) | Amano, Nagoya, Japan |
| Penicillium cyclopium (G50) | Amano, Nagoya, Japan |
| Chromobacterium viscosum (crude) | Toyo Jozo, Tokyo, Japan |
| Chromobacterium viscosum (pure) | Toyo Jozo, Tokyo, Japan |
| Rhizomucor miehei | Biocatalysts Ltd. Pontybridd, UK |
| Humicola lanuginosa | Biocatalysts Ltd. Pontybridd, UK |
| Rhizomucor miehei (Lipozyme, immob.) | Novo, Bagsvaerd, Dänemark |
| Candida antartica B (SP435, immob.) | Novo, Bagsvaerd, Dänemark |
| Candida antartica B (SP525, frei) | Novo, Bagsvaerd, Dänemark |
| Candida antartica A (SP526, frei) | Novo, Bagsvaerd, Dänemark |
| Candida antartica A,B (SP382, frei) | Novo, Bagsvaerd, Dänemark |
| Schweineleberesterase | Fluka, Buchs, Schweiz |
| Esterase aus Thermoanaerobium brockii | Fluka, Buchs, Schweiz |

Um eine Umsetzung dieser Verbindungen (II) zu ermöglichen, wurde deshalb eine Mutagenesestrategie ausgehend von einer Esterase aus Pseudomonas fluorescens, die in E. coli kloniert worden war, eingeschlagen. Die Klonierung und Sequenzierung dieser Esterase wurde zuerst von Choi et al. (Agric. Biol. Chem. Vol. 54, No. 8, 1990: 2039 - 2045) beschrieben. Pelletier und Altenbuchner (Microbiology, Vol. 141, 1995: 459 - 468) haben diese Esterase nochmals sequenziert und einige Fehler in der ursprünglichen Sequenz von Choi et al. gefunden.

Für die Mutagenese der Esterase wurde der Stamm Escherichia coli XL-1 Red (= Epicurian coli XL-1 Red) verwendet.

### 1. Herstellung der Esterase bzw. der mutierten Esterasen

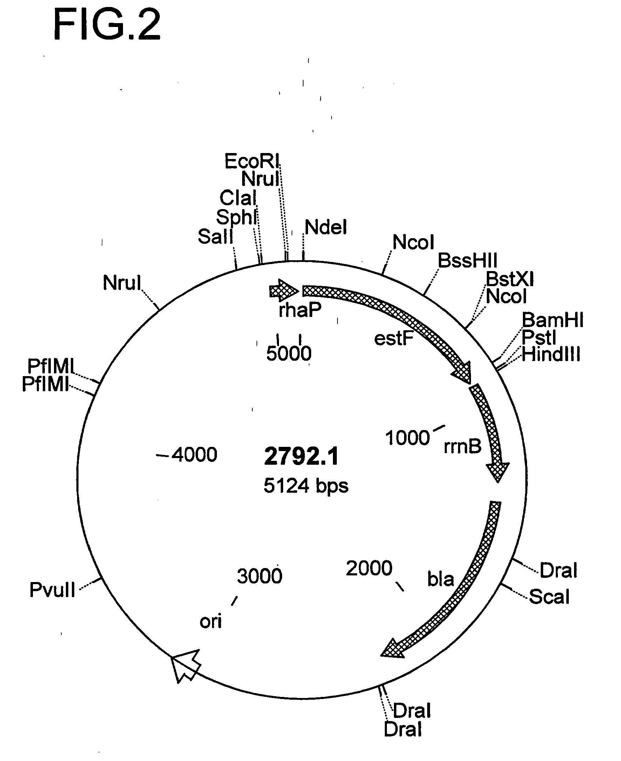

Der Stamm Escherichia coli JM109 oder DH5α, der das Rhamnose induzierbare Plasmid 2792.1 (siehe Figur 2) trägt, wurde bei 37 °C in Luria-Bertani(= LB)-Medium, das mit 100 µg/ml Ampicillin suplementiert war, bis zur frühen exponentiellen Phase (OD₆₀₀ 0,5 - 0,6, ca. 3 Stunden) angezogen. Auf dem Plasmid 2792.1 ist das Esterasegen estF lokalisiert. Die Genexpression wurde zu diesem Zeitpunkt durch Zugabe von Rhamnose [Endkonzentration 0,2 % (w/v)] zur Kultur induziert. Anschließend wurden die Zellen für weitere 3,5 Std. bei 37 °C inkubiert und über Zentrifugation (5000 rpm, 5 min, 4 °C) geerntet und zweimal mit Kaliumphosphatpuffer (50 mM, pH 7,5, 4 °C) gewaschen. Anschließend wurden die Zellen im gleichen Puffer resuspendiert und mit Ultraschall aufgeschlossen. Die Zelltrümmer wurden durch Zentrifugation (5000 rpm, 15 min, 4 °C) entfernt. Die Proteinkonzentration des Überstandes wurde mit Hilfe des Bicinchonionic acid-Proteinbestimmungskit der Firma Pierce, Rockford, Illinois, USA (Bestellnummer 23223, US 4,839,295) ermittelt. Die spezifische Aktivität der PFE wurde photometrisch oder in einem pH-Stat (siehe 2. Esteraseaktivität) bestimmt. Die PFE wurde direkt in den Hydrolyseexperimenten verwendet. Weitere Aufreinigung der PFE für die Hydrolyseexperimente erfolgte nicht, da die Esterase schon in hoher Reinheit von den Stämmen produziert wurde. Eine einfache Reinigung über eine Zinc-Affinitätschromatographie führte zu einer homogenen Esteraselösung. Dabei wurde die spezifische Aktivität nur geringfügig angehoben.

### 2. Bestimmung der Esteraseaktivität

Die Esteraseaktivität wurde in einem pH-Stat-Assay mit 5 % (w/v) Ethylacetat in einer Emulsion, die neben destilliertem Wasser 2 % (w/v) Gummi Arabicum enthält, bei 37 °C und pH 7,5 bestimmt. Zu 20 ml dieser Emulsion wurde eine bekannte Menge Esterase gegeben. Die freigesetzte Essigsäure wurde automatisch in einem pH-Stat-Automat der Firma Metrohm (Herisau, Schweiz) mit 0,1 N NaOH titriert, so daß der pH konstant blieb. Die Esteraseaktivität wurde in Unit angegeben. Unter ein Unit (= U) versteht man die Menge an Enzym, die 1 µmol Essigsäure pro Minute unter Assaybedingungen produziert. Die ermittelten Unit-Werte wurde mit dem Wert der Autolyse von Ethylacetat unter diesen Bedingungen korrigiert. Dieser Wert beträgt 0,25 µmol pro Minute bei 37 °C.

### 3. Biotransformation

Die Hydrolyse der 3-Hydroxyester wurde in Rundkolben unter Rühren (700 rpm) durchgeführt. Dazu wurden 0,5 mmol des Esters 1 oder 3 zu einem Milliliter Toluol bei 37 °C gegeben. Die Reaktion wurde durch Zugabe von 3 ml Kulturüberstand gestartet und durch Extraktion mit Ether gestoppt. Die organische Phase wurde über MgSO₄ getrocknet. Die Substrate und das Produkt wurde mit Hilfe einer Kieselgelsäule (Petrolether : Ether, 2 : 1) getrennt.

Für die Analysen der Reaktionsprodukte wurden 100 ∝1 des Reaktionsgemisches zentrifugiert und der Überstand mittels Dünnschicht (Kieselgelplatten, sichtbar machen mit UV oder ansprühen mit Cer-Reagenz) oder GC (Hewlett Packard, Model HP5890 Series II) unter Verwendung einer chiralen Säule [=Heptakis, (6-O-thexyldimethyl-silyl)-2,3-di-O-methyl)-β-cyclodextrin, 25 x 0,25 mm, 15 m, Prof. W. König, Institut für Organische Chemie, Universität Hamburg, Deutschland) analysiert. Die GC-Analyse wurde bei 125 °C (isothermal) mit Helium als Trägergas (80 kPa), einem Flammenionisationsdetektor und einem sog. "split ratio" von 1 : 100 durchgeführt.

### 4. Anzucht der Mikroorganismen

Die verwendeten Mikroorganismen (Mutagenesestamm: Escherichia coli XL-1 Red, Selektionsstamm: Escherichia coli DH5α) wurden in LB-Medium (= Luria-Bertani-Medium), LB/Amp-Medium [LB supplementiert mit Ampicillin (1 % w/v)], LB/TB/Amp-Medium [LB supplementiert mit Tributyrin (1 % v/v) und Ampicillin (1 % w/v) und 1 % Agar] und MM/Amp/Ind/Rha [minimalmedium supplementiert mit Ampicillin (1 % w/v) und als Indikatorsubstanzen Kristallviolett (1 mg/l) und Neutralrot (30 mg/L) und zur Induktion Rhamnose (0,2 % w/v) sowie 1 % Agar] angezogen. Die Zusammensetzung des LB-Mediums sowie des Minimalmediums (M9) sind Maniatis et al., Molecular Cloning: A Laboratory Manual (Sec. Edition, Vol. I, II, III, Cold Spring Habor Laboratory Press, 1989, ISBN 0-87969-309-6) oder Greener et al, (Methods in Molecular Biology, Vol. 57, 1996: 375 - 385) zu entnehmen,

### 5. Mutagenese

Die Mutagenese wurde wie in Figur 1 dargestellt durchgeführt. Das Plasmid PFE-WT wurde aus einer LB/Amp-Medium Übernachtkultur mit Hilfe eines Kits von der Firma Quiagen (Hilden, Deutschland) isoliert und anschließend zur Mutagenese in kompetente Zellen des Stammes Epicurian coli XL-1 Red transformiert und über Nacht in 50 ml LB/Amp-Medium, das mit 20 mM MgCl₂ und 20 mM Glukose supplementiert war, bei 37 °C angezogen. 500 µl dieser Kultur wurden in frisches LB/Amp-Medium (50 ml) geimpft und einem weiteren Mutationszyklus unterzogen. Von der restlichen Kultur wurden 2 ml Zellen genommen, zentrifugiert (3000 rpm, 10 min, 4 °C) und das Plasmid aus diesen Zellen isoliert (siehe Maniatis et al., Molecular Cloning: A Laboratory Manual, Sec. Edition, Vol. I, II, III, Cold Spring Habor Laboratory Press, 1989, ISBN 0-87969-309-6). Es wurden bis zu sieben Mutationszyklen nacheinander durchgeführt. Positive Mutanten, die in der ersten Runde identifiziert wurden (z.B. PFE-U3) wurden einer zweiten Mutageneserunde (bis zu sieben Zyklen) unterzogen.

### 6. Transformation der Mikroorganismen

Die Transformation der Mikroorganismen E. coli XL1 Red oder E. coli DH5α wurde nach der von Chung et al. (Proc. Natl. Acad. Sci. USA, Vol. 86, 1989: 2172 - 2175) beschriebenen Methode durchgeführt. Nach Anzucht der Transformanten über ca. 1 Stunde in LB/Amp-Medium bei 37 °C wurden Aliquots (50 - 100 µl) der Kultur auf LB/Amp-Agarplatten ausplattiert und über Nacht bei 37 °C inkubiert. Diese Platten wurden als Masterplatten für das anschließende Stempeln nach Lederberg in den Screening-Experimenten verwandt (Manual of Methods General Bacteriology, American Society For Microbiology, Washington, DC 20006, ISBN 0-914826-29-8). Zusätzlich wurden 50 µl Kultur auf LB/TB/Amp-Platten, die 0,2 % Rhamnose enthielten, zur Identifizierung von Kolonien, die eine aktive Esterase produzieren, ausplattiert.

### 7. Screening System

Zur Identifizierung der mutierten Gene wurden die Kolonien von den Masterplatten in einem Replicaplating auf folgende Selektionsplatten gestempelt (Manual of Methods for General Bacteriology, American Society For Microbiology, Washington, DC 20006, ISBN 0-914826-29-8): a) MM/Amp/Ind/Rha mit 0,1 % (w/v) Substrat (Verbindung 1), b) MM/Amp/Ind/Rha mit 0,1 % (w/v) Substrat (Verbindung 3). Die Platten wurden bei 37 °C inkubiert. Positive Klone wurden anhand der roter Färbung um und in den Kolonien - Senkung des pH-Wertes - auf den Plattentypen (a) und (b) und schnelleres Wachstum auf den (b)-Platten identifiziert. Auf LB/Amp/Ind-Platten ist bei alle Kolonien nach 2 Tagen eine Rotfärbung zu beobachten.

Dies ist vermutlich auf eine Nebenreaktion zurückzuführen. Für die Tests sind deshalb nur die oben genannten Minimalmediumsplatten geeignet. Durch Vergleich mit den Masterplatten wurden nur solche Kolonien ausgewählt, die in beiden Methoden ein positives Ergebnis lieferten, das heißt sowohl eine intakte Esterase produzierten, als auch Rotfärbung aufwiesen. Unter ca. 750 Kolonien der ersten Mutageneserunde wurden zwei positive Klone (= PFE-U1 und PFE-U3) nach 2 bis 6 Tagen Inkubation bei 37 °C identifiziert. Im Falle von PFE-U1 bildete sich nur eine schwache Rotfärbung, während im Falle von PFE-U3 sich eine starke Rotfärbung bildete und auf Verbindung 3 sich ein gutes Wachstum zeigte. Die Vektoren (= Plasmide) dieser Klone wurden isoliert und erneut in den Stamm E. coli DH5α transformiert. Diese Organismen wurden zur Produktion der mutierten Esterasen nach Rhamnoseinduktion in 250 ml LB/Amp-Medium verwendet. Die Esterase (siehe oben) wurde isoliert und zur präparativen Umsetzung der Verbindung 1 verwendet. Als Kontrolle wurde die nicht mutierte Wildtyp-Esterase verwendet.

### 8. Nucleotidsequenz-Analyse

Die Nucleotidsequenz der Esterasegene wurde unter Verwendung einer Fluoreszenz-Dideoxy-DNA-Sequenzmethode bestimmt. Die DNA-Sequenzierung wurde mit dem Taq Dye Deoxy™Cycle Sequencing Kit (Applied Biosystems, Weiterstadt, Deutschland) nach Angaben des Herstellers unter Verwendung von Primern, die von der Nucleinsäuresequenz abgeleitet wurden (Interactiva, Ulm, Germany), durchgeführt. Die Sequenzierung des PFE-UL-Gens ergab keine Mutation im Strukturgen, während die Sequenz von PFE-U3 zwei Punktmutationen aufwies. In Position 209 (A gegen D) und in Position 181 (L gegen V) fand dadurch ein Aminosäureaustausch statt. Modelling-Analysen zeigten, daß die Aminosäureaustausche weit entfernt vom Aktiven Zentrum liegen.

### 9. Chemische Synthese der Verbindungen 1 und 3

a) 5-(Benzyloxy-)-3-hydroxy-4,4-dimethylpentansäureethylester (= Verbindung 1)
   Zu einer Lösung von 170 ml Lithiumdiisopropylamid (110 mmol, hergestellt aus 11,1g Diisopropylamin und 68,8 ml Butyllithium, 1,6 N in Hexan) in THF/Hexan wurde bei - 60 °C eine Essigsäureethylesterlösung (8,8 g, 100 mmol in 10 ml THF) tropfenweise zugegeben. Nach 30 Minuten wurden 30 ml 3-Benzyloxy-2,2-dimethylpropanal (19,2 g, 100 mmol) in THF rasch zugegeben, der Ansatz 5 Minuten gerührt, bevor eine wässrige Kaliumhydrogensulfatlösung zur Neutralisierung zugegeben wurde. Der Reaktionsansatz wurde mit Diethylether extrahiert, mehrmals mit Wasser gewaschen, über Magnesiumsulfat getrocknet und das organische Lösungsmittel im Vakuum abgezogen. Es wurden 26,1 g (93 %) der Verbindung als leicht gelbes Öl isoliert. Die Verbindung wurde über eine Kieselgelsäule (Ether : Petrolether, 1:3 bis 1:1) gereinigt.
   ²H-NMR(CDCl₃) : δ = 0.90 (s,CH₃), 0,93 (s,CH₃), 1.25 (t,J = 7 Hz, CH)₃, 2.37 (dd, J = 16 Hz, J = 10 Hz, 1H), 2.49 (dd, J = 16 Hz, J = 4 Hz, 1H) , 3.28 (d, J = 9 Hz, 1 H), 3.36 (d,J = 9 Hz, 1H), 3.55 (d, J = 4 Hz, OH), 4.02 (dt, J = 10 Hz, , J = 4 Hz, 1H), 4.16 (q,J = 7 Hz, CH₂), 4.49 (s,BnCH₂), 7.30 (m, 5 Aryl-H)ppm. ¹³C-NMR(CDCl₃): δ = 14.16, 19.78, 22.10, 37.23, 38.15, 60.50, 73.42, 74.00, 78.44, 127.57, 128.34, 138.05, 173.15 ppm. IR (cap. film): v = 3500 (broad), 3064, 3028, 2976, 2936, 2904, 2872, 1732, 1476, 1452, 1368, 1308, 1256, 1184, 1156, 1096 cm⁻². MS (RT) : m/e = 280 (0,5 M⁺) , 234 (1) , 205 (1) , 190 (3), 174(1), 159 (2), 156(1), 141(4), 117(9), 111(3), 109(6), 108(13), 107(16), 91(100), 79(10), 71(12).
b) 5-(Benzyloxy-)-3-hydroxy-4,4-dimethyl-pentansäure-2',3'-dihydroxypropylester (= Verbindung 3)
   Entsprechend der unter a beschriebenen Synthese wurde unter Verwendung von 35 mmol Lithiumdiisopropylamid, 30 mmol 1-Acetyl-2,3-isopropylidenglycerin und 30 mmol 3-Benzyloxy-2,2-dimethylpropanal die Verbindung 5-(Benzyloxy-)-3-hydroxy-4,4-dimethyl-pentansäure-(2',2'-dimethyl-1,3-dioxolan)-4'-ylmethylester (= Verbindung 2) synthetisiert. Ausgehend von 3,66 g (10 mmol) dieser Verbindung in 30 ml Methoxyethanol wurde die Verbindung 3 synthetisiert. Die Lösung der Verbindung 2 wurde mit 5,0 g Borsäure auf 110 °C für 20 Minuten erhitzt. Nach Entfernung des Lösungsmittels wurde der Rückstand mit geringen Mengen an Wasser versetzt und mit Diethylether extrahiert. Der Extrakt wurde über Magnesiumsulfat getrocknet, das Lösungsmittel im Vakuum abgezogen und die Verbindung 3 über eine Kieselgelchromatographie (Ether und Ether : Dioxan, 9 : 1) gereinigt. 2,3 g (70 %) der Verbindung 3 wurden als farbloses Ö1 in einem 1 : 1 Diastereomerengemisch isoliert.
   ¹H-NMR(CD₃OD): δ = 0.90 (S, CH₃), 0,92 (s,CH₃), 2.40 (m, 1H), 2.59 (m, 1H), 3.24 (d, J = 9 Hz, 1 H), 3.33 (d, J = 9 Hz, 1H), 3.56 (m, CH₂), 3.84 (quint, J = 5 Hz, 1H), 3.99 - 4.22 (sev. m, 3H), 4.45 (d,J = 12 Hz, 1H)), 4.51 (d,J = 12 Hz, 1H) 7.31 (m, 5 Aryl-H)ppm. ¹³C-NMR(CD₃OD): δ = 21.00, 21.63, 38.52, 39.82, 64.00, 64.02, 66.64, 66.68, 71.08, 71.10, 74.07, 74.35, 78.21, 128.56, 128.67, 129.35, 139.94, 174.41 ppm. IR (CHCl₃): v = 3600 (broad), 3436 (broad), 3088, 3064, 3000, 2964, 2876, 1732, 1600, 1452, 1412, 1384, 1364, 1308, 1284, 1256, 1228, 1180, 1156, 1088, 1052 cm⁻¹. MS (140 °C): m/e = 326 (0.5, M⁺), 308 (1), 235 (4), 202 (5), 188 (3), 174 (3), 163(9), 145(19), 108(19), 107(11), 91(100), 71(12).

### 10. Enzymatische Hydrolyse mit PFE-U3

Die enzymatische Hydrolyse von der racemischen Verbindung 1 mit der mutierten Esterase PFE-U3 führte zur korrespondierenden Säure [α]_{D}²⁰= - 11,01° (c = 0,965, CHCl₃), Öl, 11,6 % y und dem verbliebenen Startmaterial (24,9 %ee), [α]_{D}²⁰= + 0,97° (c = 3,071, CHCl₃), Öl, 61,4 % y. Die Hydrolyse erfolgte also mit einer stereoselektiven Diskriminierung von 1. Die Wildtyphydrolase als Kontrolle ebenso wie die Mutante PFE-U1 ist nicht in der Lage die Verbindung 1 umzusetzen. Mutanten der zweiten Generation ausgehend von PFE-U3 zeigten keine Verbesserung in der Stereoselektivität in der Hydrolysereaktion. Mutanten der zweiten Generation sind PFE-3-311 bis PFE-3-713 (siehe Tabelle II). Tabelle II gibt die spezifische Aktivität der verschiedenen Esterasen in Units mit Essigsäureethylester als Substrat wieder (Tabelle II, U/mg Protein). Die optische Aktivität der verschiedenen Esterasen bezieht sich auf die Verbindung 1 (siehe Tabelle II).

**Tabelle II: Substratspezifität verschiedener Esterasen**

| Esterase | Aktivität (U/mg) gegen Ethylacetat | Enantiomerenüberschuß (%ee bezogen auf Verbindung 1) |
|---|---|---|
| PFE-WT | 6,0 | 0 |
| PFE-U1 | 4,9 | 0 |
| PFE-U3 | 2,3 | 24,9 |
| PFE-U3-311 | 0,9 | 3,3 |
| PFE-U3-411 | 1,3 | 7,0 |
| PFE-U3-412 | 2,1 | 3,7 |
| PFE-U3-413 | 2,5 | 3,5 |
| PFE-U3-511 | 0,9 | 3,3 |
| PFE-U3-512 | 4,4 | 3,1 |
| PFE-U3-513 | 1,5 | 3,3 |
| PFE-U3-514 | 4,1 | 4,3 |
| PFE-U3-515 | 1,1 | 3,7 |
| PFE-U3-711 | 2,7 | 3,4 |
| PFE-U3-712 | 2,3 | 3,2 |
| PFE-U3-713 | 1,3 | 3,2 |

## Patentansprüche

1. Verfahren zur Änderung der Substratspezifität von Enzymen, **dadurch gekennzeichnet, daß** man folgende Arbeitsschritte durchführt:
a) Einbringen einer DNA, die eine Kopie des für das Enzym kodierenden Gens enthält, in den Escherichia coli Stamm XL1 Red oder in ein funktionelles Derivat, das die Genmarker relAl, mutS, mutT und mutD5 trägt,
b) Inkubation des transformierten Escherichia coli Stammes XL1 Red oder seines funktionellen Derivat zur Erzeugung von Mutationen im Enzymgen,
c) Weitergabe der mutierten DNA aus dem Stamm XL1 Red oder seinem funktionellen Derivat an einen Mikroorganismus, der keine behindernde Enzymaktivität aufweist,
d) Inkubation dieses Mikroorganismus zur Detektion der Enzymaktivität auf oder in mindestens einem Selektionsmedium, das mindestens ein Enzymsubstrat, das die Erkennung einer geänderten Substratspezifität des Enzyms ermöglicht, und ggf. weitere Indikatorsubstanzen enthält,
e) Auswahl der Mikroorganismen, die eine Änderung der Substratspezifität zeigen,
wobei die Änderung der Substratspezifität zu einer stereoselektiven enzymatischen Aktivität führt.

2. Verfahren nach Anspruch 1, **dadurch gekennzeichnet, daß** man die Arbeitsschritte (a) bis (e) mehrmals in einer Folge durchläuft, indem man die DNA aus den unter Schritt (e) ausgewählten Mikroorganismen wieder in den Stamm Escherichia coli XL1-Red oder seinem funktionellen Derivat zurückbringt.

3. Verfahren nach Anspruch 1 oder 2, **dadurch gekennzeichnet, daß** als Mikroorganismen prokaryontische oder eukaryontische Mikroorganismen verwendet werden.

4. Verfahren nach den Ansprüchen 1 bis 3, **dadurch gekennzeichnet, daß** als Mikroorganismen grampositive oder gramnegative Bakterien, Pilze oder Hefen verwendet werden.

5. Verfahren nach den Ansprüchen 1 bis 4, **dadurch gekennzeichnet, daß** als Enzym eine Hydrolase verwendet wird.

6. Verfahren nach den Ansprüchen 1 bis 5, **dadurch gekennzeichnet, daß** als Enzym eine Hydrolase ausgewählt aus der Gruppe bestehend aus Proteasen, Lipasen, Phospholipasen, Esterasen, Phosphatasen, Amidasen, Nitrilasen, Etherhydrolasen, Peroxidasen und Glykosidasen verwendet wird.

7. Verfahren nach den Ansprüchen 1 bis 6, **dadurch gekennzeichnet, daß** man eine Lipase, Esterase, Nitrilase oder Phytase verwendet.

## Claims

1. A method for altering the substrate specificity of enzymes, which comprises carrying out the following steps:
a) introducing a DNA which comprises a copy of the gene coding for the enzyme into the Escherichia coli strain XL1 Red or into a functional derivative which carries the genetic markers relAl, mutS, mutT and mutD5,
b) incubating the transformed Escherichia coli strain XL1 Red or its functional derivative to generate mutations in the enzyme gene,
c) transmitting the mutated DNA from the strain XL1 Red or its functional derivative to a microorganism which has no impeding enzyme activity,
d) incubating this microorganism to detect the enzyme activity on or in at least one selection medium which comprises at least one enzyme substrate which makes it possible to recognize an altered substrate specificity of the enzyme, and, if appropriate, other indicator substances,
e) selecting the microorganisms which show an alteration in the substrate specificity, where the alteration in the substrate specifically results in a stereoselective enzymatic activity.

2. The method according to claim 1, wherein steps (a) to (e) are performed several times in sequence by returning the DNA from the microorganisms selected in step (e) to the strain Escherichia coli XL1-Red or its functional derivative.

3. The method according to claim 1 or 2, wherein prokaryotic or eukaryotic microorganisms are used as microorganisms.

4. The method according to any of claims 1 to 3, wherein Gram-positive or Gram-negative bacteria, fungi or yeasts are used as microorganisms.

5. The method according to any of claims 1 to 4, wherein a hydrolase is used as enzyme.

6. The method according to any of claims 1 to 5, wherein a hydrolase selected from the group consisting of proteases, lipases, phospholipases, esterases, phosphatases, amidases, nitrilases, ether hydrolases, peroxidases and glycosidases is used as enzyme.

7. The method according to any of claims 1 to 6, wherein a lipase, esterase, nitrilase or phytase is used.

## Revendications

1. Procédé de modification de la spécificité d'enzymes pour un substrat, **caractérisé en ce qu'**on effectue les étapes suivantes :
a) une introduction d'un ADN, qui contient une copie du gène codant pour l'enzyme, dans la souche XL1 Red d'Escherichia coli ou dans un dérivé fonctionnel qui porte les marqueurs génétiques relAl, mutS, mutT et mutD5,
b) une incubation de la souche XL1 Red d'Escherichia coli transformée ou de son dérivé fonctionnel pour la production de mutations dans le gène enzymatique,
c) un transfert de l'ADN muté issu de la souche XL1 Red ou de son dérivé fonctionnel sur un micro-organisme qui ne présente pas d'activité enzymatique entravante,
d) une incubation de ce micro-organisme pour la détection de l'activité enzymatique sur ou dans au moins un milieu de sélection qui contient au moins un substrat, qui permet la reconnaissance d'une modification de spécificité de l'enzyme pour le substrat, et éventuellement d'autres substances indicatrices,
e) une sélection des micro-organismes qui montrent une modification de la spécificité pour le substrat,
la modification de la spécificité pour le substrat donnant lieu à une activité enzymatique stéréosélective.

2. Procédé suivant la revendication 1, **caractérisé en ce qu'**on parcourt en succession les étapes (a) à (e) à plusieurs reprises, en ramenant l'ADN issu des micro-organismes sélectionnés dans l'étape (e) à nouveau dans la souche Escherichia coli XL1 Red ou son dérivé fonctionnel.

3. Procédé suivant la revendication 1 ou 2, **caractérisé en ce que**, comme micro-organismes, on utilise des micro-organismes procaryotes ou eucaryotes.

4. Procédé suivant les revendications 1 à 3, **caractérisé en ce que**, comme micro-organismes, on utilise des bactéries gram-positives ou gram-négatives, des champignons ou des levures.

5. Procédé suivant les revendications 1 à 4, **caractérisé en ce que**, comme enzyme, on utilise une hydrolase.

6. Procédé suivant les revendications 1 à 5, **caractérisé en ce que**, comme enzyme, on utilise une hydrolase choisie parmi le groupe constitué des protéases, des lipases, des phospholipases, des estérases, des phosphatases, des amidases, des nitrilases, des étherhydrolases, des peroxydases et des glycosidases.

7. Procédé suivant les revendications 1 à 6, **caractérisé en ce qu'**on utilise une lipase, estérase, nitrilase ou phytase.
